(19) Europäisches Patentamt
European Patent Office
Office européen des brevets

(11) Publication number: **0 034 425**
**B1**

(12) **EUROPEAN PATENT SPECIFICATION**

(45) Date of publication of patent specification: **16.05.84**

(21) Application number: **81300388.6**

(22) Date of filing: **30.01.81**

(51) Int. Cl.$^3$: **C 07 C 85/04,** C 07 C 87/50, C 07 C 87/64, C 07 C 87/58, C 07 D 209/88, C 08 G 73/02

Bridgewater New Jersey 08807 (US)

(54) **Process for preparing arylamines.**

(30) Priority: **04.02.80 US 118147**
**12.12.80 US 215610**

(43) Date of publication of application:
**26.08.81 Bulletin 81/34**

(45) Publication of the grant of the patent:
**16.05.84 Bulletin 84/20**

(84) Designated Contracting States:
**DE FR GB NL**

(56) References cited:
**US-A-3 314 788**

**Chemical abstracts vol. 78, no. 7, 1973 Columbus, Ohio, USA S. CREASON et al. "Electrochemical and spectroscopic studies of cation radicals. I. Coupling rates of 4-substituted triphenylaminium ion" pages 403, 404, abstract no. 42409f**

**Chemical Abstracts vol. 70, no. 11, 1969 Columbus, Ohio, USA I.E. MOISAK et al. "Tertiary aromatic amines" page 289, abstract no. 47064j**

(73) Proprietor: **XEROX CORPORATION**
**Xerox Square - 020**
**Rochester New York 14644 (US)**

(72) Inventor: **Turner, Richard S.**
**312 Eileen Way**
**Bridgewater New Jersey 08807 (US)**
Inventor: **Renfer, Dale S.**
**30 Kansas Street**
**Rochester New York 14609 (US)**
Inventor: **Yanus, John F.**
**924 Little Bardfield Road**
**Webster New York 14580 (US)**

(74) Representative: **De Minvielle-Devaux, Ian Benedict Peter**
**CARPMAELS & RANSFORD 43, Bloomsbury Square**
**London WC1A 2RA (GB)**

**EP 0 034 425 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a process of processing a tertiary amine by condensation of a mono- or di-secondary amine and a mono- or di-iodoaryl compound under certain conditions, and to the resulting products.

This invention provides an improved chemical process for the preparation of this class of tertiary amines. The reaction involved can be classified as a particular form of the Ullman condensation reaction. The reaction is between an aryliodide and an arylamine. In other words, the reaction involves the arylation of a secondary amine. The resulting product is either a non-polymeric compound or a polymeric compound, depending upon the starting materials used.

The chemical literature shows that the most useful synthesis of arylamines involves the coupling of a diarylamine and a haloaromatic compound, preferably an iodoaromatic compound, in the presence of a base and copper in a polar solvent. The reaction which led to this synthesis was discovered by F. Ullmann (Chem. Ber. *36*, 2382 (1903)). This reaction is difficult and possesses many undesirable features. Normally it is necessary to use a high temperature reaction, i.e. at over 200°C in a polar solvent with a copper catalyst and $K_2CO_3$ base to achieve arylation at a reasonable rate. In an analogous reaction, not of the kind to which the present invention relates (S.C. Creason, et al., J. Org. Chem., *37*, 4440 (1972)), a substituted aniline is reacted with iodobenzene at about 200°C; potassium carbonate and copper are employed without a solvent. These processes require too high a temperature which introduces lack of control and difficulties in operating the processes. The intended products of these reactions are obtained in comparatively poor yields.

In U.S. Patent 3,314,788, there is disclosed the reaction of a primary aryl diamine and an alkyl halide (see column 2, lines 39—47). This reaction is distinguished from the reaction of a secondary diarylamine and an aryhl halide. This latter reaction is said to be difficult and expensive and yields phenyl instead of benzyl derivatives. This reaction, which is of the type with which the present invention is concerned, is no longer difficult and expensive if it is conducted in accordance with the present invention.

It is an object of the present invention to provide a process which enables arylamines to be prepared in a relatively easy manner and in comparatively high yields.

The invention provides a process of preparing a tertiary amine by the condensation of a mono- or di-secondary amine and a mono- or di-iodoaryl compound, characterised in that the condensation reaction is conducted in the presence of potassium hydroxide and a copper catalyst, and either in the absence of a solvent or with an inert saturated hydrocarbon solvent, in an inert atmosphere, at a temperature from 120°C to 190°C for a period of time sufficient to at least substantially complete the reaction.

Preferred mono-secondary amines have the general formula $R_2R_3NH$ wherein $R_2$ and $R_3$ (which may be the same or different) are alkyl, alkenyl, aryl, alkaryl or aralkyl. Examples of these amines are: 3-methyldiphenylamine, diphenylamine and diethylamine.

Preferred di-secondary amines have the general formula $R_4HN—R_1—NHR_4$ wherein $R_1$ is divalent arylene or alkylidene and $R_4$ is aryl. Examples of these amines are N,N'-diphenyl - [1,1'-biphenyl] - 4,4' - diamine; N,N' - diphenyl - [phenylene] - 1,4 - diamine; N,N' - diphenyl - [p,p'' - terphenyl] - 4,4'' - diamine; N,N'-diamine; N,N' - diphenyl - [p,p'' - terphenyl] - 4,4'' - diamine; N,N' - diphenyl - [p,p''' - quatraphenyl] - 4,4''' - diamine, and 4,4'-isopropylidene bis(diphenylamine).

Examples of mono- and di-iodoaryl compounds are: 4,4'-di-iodobiphenyl; 1,-4-diiodobenzene; 4,4''-diiodoterphenyl; 1,6-diiodopyrene; 3,6-diiodo-N-ethyl-carbazole; 4,4'''-diiodoquatraphenyl; 2,2-bis(4-iodophenyl) propane; iodobenzene; and p-iodotoluene.

It is obvious that when one of the reactants is monofunctional the resulting tertiary amine will be non-polymeric. When the reactants are both difunctional a polymeric polytertiary amine will result.

The condensation reaction is conducted in the presence of potassium hydroxide and a copper catalyst (generally in power form) either in the absence of a solvent or with an inert saturated hydrocarbon solvent, in an inert atmosphere at a temperature from 120°C to 190°C for a period of time sufficient to substantially complete the reaction.

In prior art reactions for producing similar products to those of the present invention, significant variations in catalytic effect were observed when different copper catalysts were employed with potassium carbonate as the base. The use of KOH as the base has been found significantly to mitigate these variations. The mitigation is so great that it has been found that any finely divided copper catalyst can be employed so long as potassium hydroxide is employed as the base. Examples of copper catalysts are: metallic copper powder, cupric oxide, cuprous oxide, cuprous sulfate and cuprous sulfide. In fact, any copper catalyst heretofore commonly used in the Ullman condensation reaction can be employed. In addition, the use of KOH allows shorter reaction times and lower reaction temperatures.

It has been found that an inert atmosphere is necessary for obtaining the intended product of the process. The inert atmosphere should be as inert to the reaction as is practicable. Examples of gases providing an inert atmosphere are argon, nitrogen and methane. The inert atmosphere should be present at the beginning of the reaction and, in particular, by the time the amine component is introduced into the reaction system.

In this reaction the ratio of base to amine should be such that the base is present as an excess in relation to the amine. The molar ratio can generally range from 1.5 to 1 to 6 to 1.

As indicated above, the use of KOH allows significant reduction in the reaction temperatures as compared to prior art reactions employing $K_2CO_3$ as the base. The reaction temperature range can be from 120°C to 190°C, with the preferred reaction temperature being from 135 to 165°C. At temperatures lower than about 125°C, the reaction does not proceed at a practical rate because the KOH/Cu does not form a melt until this temperature.

The process of the present invention can be carried out in the absence of a solvent when the intended product is very soluble at ambient temperature in the inert hydrocarbon solvent. When the intended product is at least relatively insoluble at ambient temperature in the inert hydrocarbon solvent, the use of KOH yields a relatively pure product which can be further highly purified by recrystallisation from the same solvent. Aprotic or polar solvents cannot be employed without formation of interfering and yield-reducing by-products.

An advantage of the present process is the fact that relatively pure product can be obtained via the present process when KOH and an inert hydrocarbon solvent system is employed. This is distinguished from employing an aprotic or polar solvent. A further advantage gained from the use of an inert high boiling hydrocarbon solvent lies in the fact that the intended reaction product can be purified from the same solvent. This eliminates difficult handling conditions when a different solvent or purification means is employed.

Any commercially available KOH in flake or pellet form with a low water content can be employed. The flake form is preferred.

The inert hydrocarbon solvent is normally an aliphatic hydrocarbon having an initial boiling point above 170°C., for example dodecane or tetradecane.

Particularly preferred solvents are mixtures of $C_{13}$—$C_{15}$ aliphatic hydrocarbons, for example Soltrol 170 (initial b.p. 218°C), and Soltrol 130 (initial b.p. 176°C) available from Phillips Chemical Company. "Soltrol" is a Trade Mark.

The following examples are non-limiting illustrations of the present invention.

Example I

Preparation of N,N'-diphenyl-N,N'-bis-(3-methylphenyl)-[1,1'-biphenyl]-4,4'-diamine having the following structural formula:

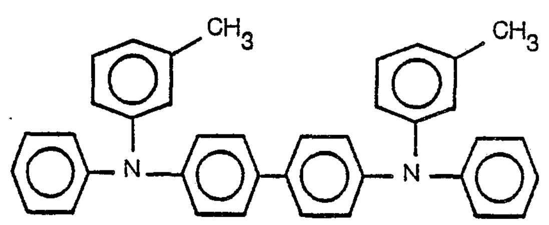

Into a 250 ml, three necked round bottom flask equipped with a mechanical stirrer and a thermometer with temperature controller and purged with argon were placed 8.1 grams diodobiphenyl (0.02 mole), 14.6 grams 3-methyldiohenylamine (0.08 mole), 9 grams potassium hydroxide flake (0.16 mole), 6 grams copper powder and 12 ml Soltrol 170 (a mixture of $C_{13}$—$C_{15}$ aliphatic hydrocarbons from Phillips Chemical Company). The system was maintained under this inert atmosphere throughout the reaction. The contents were heated to 160°C for about 5 hours with moderate stirring. The product was isolated by the addition of 150 ml Soltrol 170 and hot filtration (at about 140°C) to remove inorganic solids. The yellow filtrate was cooled with stirring, yielding a yellow solid. The yellow solid was dissolved in toluene and column chromatographed used Woelm neutral alumina with toluene as eluent. A colourless solid was recovered from the mother liquors and recrystallised from n-octane to yield colourless crystals of the intended product, melting point 167—168°C, with a yield of 85%.

Example II

Preparation of N,N'-diphenyl-N,N'-bis(3-methylphenyl)-(p-terphenyl)-4,4''-diamine having the following structural formula:

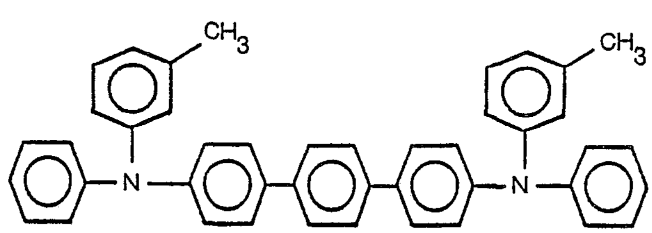

The same equipment and conditions as in Example I were employed with the following charge: 9.6 grams (0.02 mole) diiodoterphenyl, 14.6 grams (0.08 mole) 3-methyldiphenylamine; 9.0 grams (0.16 mole) potassiium hydroxide flake, 6.0 grams copper powder, 12.0 ml Soltrol 170. The above-identified intended product was obtained as colourless crystals having a melting point 188—190°C. The yield was 75%.

Example III

Preparation of N,N′-diphenyl-N,N′-bis[3-methylphenyl]-pyrenyl-1,-6-diamine having the following structural formula:

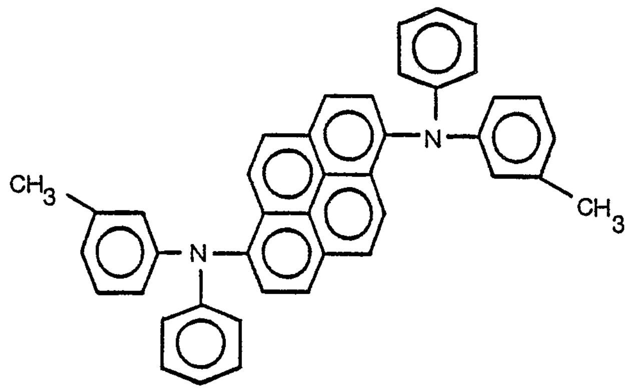

The same equipment and conditions as in Example I were employed with the following charge: 9.1 grams (0.02 mole) 1,6-diiodopyrene, 14.6 grams (0.08 mole) 3-methyldiphenylamine, 9.0 grams (0.16 mole) potassium hydroxide flake, 6.0 grams copper powder and 12.0 ml Soltrol 170. After column chromatographing the green filtrate using toluene as the eluent, the resulting deep yellow solid was extracted with acetone to yield yellow crystals of the intended compound having a melting point of 236°—238°C. The yield was 75%.

Example IV

Preparation of N-ethyl-2,7-bis[N′-phenyl-N′-(3-methylphenyl)-amino] carbazole having the following structural formula:

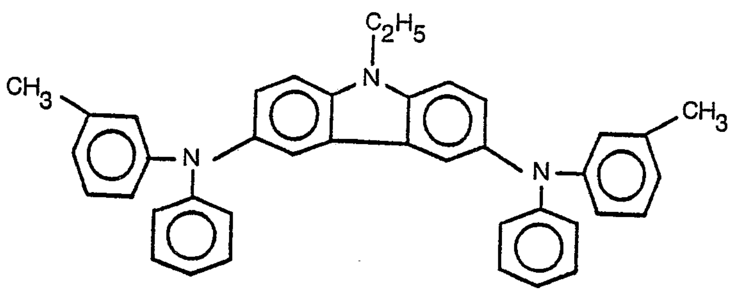

The same equipment and conditions as in Example I were employed with the following charge: 8.4 grams (0.02 mole) 3,6-diiodo-N-ethylcarbazole, 14.0 grams (0.08 mole) 3-methyl-diphenylamine, 9.0 grams (0.16 mole) potassium hydroxide flake, 6.0 grams copper powder and 12.0 ml Soltrol 170. The above-identified product was obtained as a colourless solid having a melting point of 195°—197°C. The yield was 72%.

Example V

Preparation of N,N′-diphenyl-N,N′-bis-(3-methylphenyl)-[1,1′-biphenyl]-4,4′-diamine having the structural formula in Example I in the absence of the aliphatic hydrocarbon solvent.

A 500 ml 3 necked round bottom flask equipped with an argon purge, a condenser and an overhead mechanical stirrer was charged with 81.2 grams (0.2 mole) 4,4″-diiodobiphenyl, 146.4 grams (0.8 mole) 3-methyldiphenylamine, 89.6 grams (1.6 mole) KOH flake and 80 grams (1.0 mole) copper powder. The flask was immersed in a 165°C oil bath and the two-phase melt was stirred for 3 hours. Thereafter, hot (140°C) Soltrol 170 was added and the inorganic solid separated by vacuum filtration. On cooling, the product cyrstallised from the filtrate and was isolated in 89% yield by filtration. Purification was accomplished by slurrying the product with neutral alumina (10 grams) in 1 litre of Soltrol 170 at 150°C for six hours. The alumina was removed by filtration and the purified product crystallised from the filtrate on cooling. Isolation by filtration was accomplished with a 95% recovery of the product.

Example VI

Preparation of N,N'-diphenyl-N,N'-bis(3-methylphenyl)-1, 4-phenylenediamine having the following structural formula:

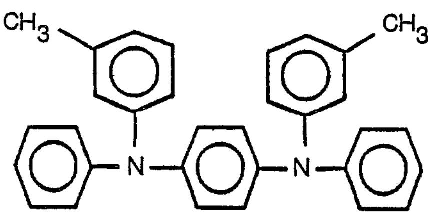

The same equipment and conditions as in Example I were employed with the following charge: 6.6 grams (0.02 mole) 1,4-diiodobenzene, 14.6 grams (0.08 mole) 3-methyldiphenylamine, 9.0 grams (0.16 mole) potassium hydroxide, 6.0 grams copper powder and 12.0 ml Soltrol 170. The above-identified intended product was obtained as a colourless product having a melting point of 195°—197°C. The yield was 81%.

Example VII

Preparation of triphenylamine.

The same equipment and conditions as in Example I were employed with the following charge: 20.4 grams (0.1 mole) iodobenzene, 27.4 grams (0.15 mole) diphenylamine, 16.8 grams (0.3 mole) potassium hydroxide flakes, 15.0 grams copper powder and 30.0 ml Soltrol 170. The above-identified product was obtained as colourless crystals having a melting-point of 125°—126°C. The yield was 82%.

Example VIII

Preparation of 3-methylphenyldiphenylamine.

The same equipment and conditions as in Example I were employed with the following charge: 20.4 grams (0.1 mole) iodobenzene, 27.5 grams (0.15 mole) 3-methyldiphenylamine, 15.0 grams copper powder and 30.0 ml Soltrol 170. The above-identified intended product was obatined as colourless crystals having a melting point of 69°—70°C. The yield was 75%.

Example IX

This example relates to the preparation of the polymer resulting from the condensation of 4,4'-isopropylidene bis (diphenylamine) and 4,4'-diiodobiphenyl.

In a 100 ml three-necked round bottom flask equipped with a mechanical stirrer and purged with argon were placed 9.3 grams (0.025 mole) 4,4'-isopropylidene bis(diphenylamine), 16.9 grams (0.3 mole) potassium hydroxide flakes, 7.5 grams copper powder and 25 ml tetrahydronaphthalene. With the aid of an oil bath, the mixture was heated to 170°C with stirring for one hour. The 4,4'-diiodobiphenyl, in an amount of 10.3 grams (0.025 mole), was added and the heterogeneous mixture was allowed to be stirred for 18 hours. The reaction was cooled and 25 ml tetrahydrofuran was added and brought to reflux. The liquid portion was decanted and the tetrahydrofuran reflux wash was repeated three times. The resulting solution was filtered and a fine yellow precipitate was formed upon addition to one litre of ethanol. The precipitate was dissolved in toluene and precipitated into acetone. This toluene-acetone sequence was repeated. The product was again dissolved in toluene and column chromatographed on Florisil, i.e. a magnesium silicate. The colourless eluent was precipitated into ethanol and dried to yield (65%) of a colourless powder. This polymer had $\bar{M}n$ of 10,000 and $\bar{M}w$ greater than 20,000 as determined from a gel permeation chromatography analysis and vapour phase osmometry.

Example X

This example relates to the preparation of the polymer resulting from the condensation of N,N'-diphenyl[1,1'-biphenyl]-4,4'-diamine and 1,4-diiodobenzene.

In a 100 ml, three-necked round bottom flask equipped with a mechanical stirrer and purged with argon is placed 3.36 grams (0.01 mole) N,N'-diphenyl-[1,1'-biphenyl]-4,4'-diamine, 4.5 grams potassium hydroxide flakes, 3.0 grams copper powder and 30 ml tetrahydronaphthalene. With the aid of an oil bath, the mixture was heated to 150°C for one hour. The 1,4-diiodobenzene, in an amount of 3.29 grams (0.01 mole), was added and the heterogeneous mixture was stirred at 150°C for 3 to 4 hours. The mixture was filtered while hot. 25 ml tetrahydrofuran were added and the mixture was brought to reflux. The tetrahydrofuran reflux wash was repeated three times. The resulting solution was

filtered and a precipitate formed by the addition of a litre of methanol. The precipitate was isolated and purified to give a yield of 4.0 grams of the polymer.

**Claims**

1. A process of preparing a tertiary amine by the condensation of a mono- or disecondary amine and a mono- or di-iodoaryl compound, characterised in that the condensation reaction is conducted in the presence of potassium hydroxide and a copper catalyst, and either in the absence of a solvent or with an inert saturated hydrocarbon solvent, in an inert atmosphere, at a temperature from 120°C to 190°C for a period of time sufficient to at least substantially complete the reaction.

2. The process of claim 1 wherein the copper catalyst is copper powder.

3. The process of claim 1 or 2 wherein the reaction is carried out at a temperature from 135°C to 165°C.

4. The process of any of claims 1—3 wherein the reaction is carried out in the presence of a solvent which is an aliphatic hydrocarbon having a boiling point above 170°C.

5. The process of claim 4 wherein the solvent is a mixture of $C_{13}$—$C_{15}$ aliphatic hydrocarbons.

6. The process of any of claims 1—3 wherein the reaction is carried out in the absence of a solvent.

7. The process of any of claims 1—6, wherein the potassium hydroxide is employed in flake form.

8. The process of any of claims 1—7 wherein the molar ratio of base to amine is from 1.5:1 to 6:1.

9. The process of any of claims 1—8 wherein there is employed a mono-secondary amine having the general formula $R_2R_3NH$ wherein $R_2$ and $R_3$ (which may be the same or different) are alkyl, alkenyl, aryl, alkaryl or aralkyl, or a di-secondary amine having the general formula $R_4HN—R_1HR_4$ wherein $R_1$ is divalent arylene or alkylidene and $R_4$ is aryl.

**Patentansprüche**

1. Verfahren zur Herstellung eines tertiären Amins durch Kondensation eines mono- oder disekundären Amins und einer Mono- oder Di-Jodarylverbindung, dadurch gekennzeichnet, daß die Kondensationsreaktion in Gegenwart von Kaliumhydroxid und eines Kupfer-Katalysators und entweder in Abwesenheit eines Lösungsmittels oder mit einem inerten gesättigten Kohlenwasserstoff-Lösungsmittel, in einer inerten Atmosphäre bei einer Temperatur von 120 bis 190°C über einen Zeitraum, der ausreicht, um die Reaktion mindestens überwiegend zu vervollständigen, ausgeführt wird.

2. Verfahren nach Anspruch 1, dadurch gekennzeichnet, daß der Kupfer-Katalysator aus Kupfer-Pulver besteht.

3. Verfahren nach Anspruch 1 oder 2, dadurch gekennzeichnet, daß die Reaktion bei einer Temperatur von 135 bis 165°C ausgeführtt wird.

4. Verfahren nach mindestens einem der Ansprüche 1 bis 3, dadurch gekennzeichnet, daß die Reaktion in Gegenwart eines Lösungsmittels, das ein aliphatischer Kohlenwasserstoff mit einem Siedepunkt oberhalb 170°C ist, ausgeführt wird.

5. Verfahren nach Anspruch 4, dadurch gekennzeichnet, daß das Lösungsmittel eine Mischung aus $C_{13}$—$C_{15}$-aliphatischen Kohlenwasserstoffen ist.

6. Verfahren nach mindestens einem der Ansprüche 1 bis 3, dadurch gekennzeichnet, daß die Reaktion in Abwesenheit eines Lösungsmittels ausgeführt wird.

7. Verfahren nach mindestens einem der Ansprüche 1 bis 6, dadurch gekennzeichnet, daß das Kaliumhydroxid in Blättchenform eingesetzt wird.

8. Verfahren nach mindestens einem der Ansprüche 1 bis 7, dadurch gekennzeichnet, daß das Molverhältnis von Base zu Amin 1,5:1 bis 6:1 beträgt.

9. Verfahren nach mindestens einem der Ansprüche 1 bis 8, dadurch gekennzeichnet, daß ein mono-sekundäres Amin der allgemeinen Formel $R_2R_3NH$ eingesetzt wird, wobei $R_2$ und $R_3$ (die gleich oder voneinander verschieden sein können) Alkyl, Akenyl, Aryl, Alkaryl oder Aralkyl bedeuten, oder daß ein di-sekundäres Amin der allgemeinen Formel $R_4HN—R_1HR_4$ eingesetzt wird, worin $R_1$ divalentes Arylen oder Alkyliden und $R_4$ Aryl bedeuten.

**Revendications**

1. Procédé de préparation d'une amine tertiaire par la condensation d'une mono- ou di-amine secondaire et d'un composé mono- ou di-iodoarylique, caractérisé en ce que la réaction des condensation est conduite en présente de potasse et d'un catalyseur au cuivre, et en l'absence d'un solvant ou avec un solvant hydrocarboné saturé inerte, sous un atmosphère inerte, à une température de 120°C à 190°C pendant une période de temps suffisante pour achever au moins sensiblement la réaction.

2. Procédé selon la revendication 1, dans lequel le catalyseur au cuivre est de la poudre de cuivre.

3. Procédé selon la revendication 1 ou 2, dans lequel la réaction est réalisée à une température de 135°C à 165°C.

4. Procédé selon l'une quelconque des revendications 1 à 3 dans lequel la réaction est réalisée en présence d'un solvant qui est un hydrocarbure aliphatique ayant un point d'ébullition au-dessus de 170°C.

5. Procédé selon la revendication 4, dans lequel le solvant est un mélange d'hydrocarbures aliphatiques en $C_{13}$—$C_{15}$.

6. Procédé selon l'une quelconque des revendications 1 à 3, dans lequel la réaction est réalisée en l'absence d'un solvant.

7. Procédé selon l'une quelconque des revendications 1—6, dans lequel la potasse est employée sous forme de paillettes.

8. Procédé selon l'une quelconque des revendications 1—7, dans lequel le rapport molaire base/amine est de 1,5:1 à 6:1.

9. Procédé selon l'une quelconque des revendications 1—8 dans lequel on emploie une monoamine secondaire ayant la formule générale $R_2R_3NH$ où $R_2$ et $R_3$ (qui peuvent être les mêmes ou différents) représentent un groupe alkyle, alkényle, aryle, alkaryle ou aralkyle, ou une di-amine secondaire ayant la formule générale $R_4HN—R_1HR_4$ où $R_1$ est un groupe arylène ou alkylidène divalent et $R_4$ est un groupe aryle.